# EUROPEAN PATENT APPLICATION

(11) **EP 4 059 354 A1**
(43) Date of publication of application: **21.09.2022**
(21) Application number: 20887296.0
(22) Date of filing: 16.11.2020
(51) Int. Cl.: A23L 33/135, A61K 35/745, A23C 9/16, C12P 7/52

(54) **SYNERGISTIC COMBINATION OF BUTYRIC-ACID-PRODUCING PREBIOTICS AND PROBIOTICS**

(30) Priority: 15.11.2019 CN 201911115800
(71) Applicant: Inner Mongolia Yili Industrial Group Co., Ltd., Inner Mongolia 010000 (CN)
(72) Inventor: SHI, Yujie, Hohhot, Inner Mongolia 010000 (CN); LIU, Biao, Hohhot, Inner Mongolia 010000 (CN)
(74) Representative: Kolster Oy Ab
(86) International application number: PCT/CN2020/128922
(87) International publication number: WO 2021/093880

(57) **Abstract**

Provided is a synergistic combination of butyric-acid-producing prebiotics and probiotics, which is used in the fields of foods, dietary supplements and drugs. Specifically, the present invention relates to a composition containing probiotics and prebiotics, a food, dietary supplement or pharmaceutical preparation comprising the composition, and the use of the composition for alleviating and improving a disease or a discomfort, or reducing the occurrence of a disease or a discomfort, wherein the disease or discomfort is related to insufficient butyric acid in the intestine, or to insufficient butyric-acid-producing microorganisms in the intestine. The present invention further relates to a method for promoting *Bifidobacterium lactis* HN019 to produce butyric acid, the method comprising using a galactooligosaccharide and lactulose as carbon sources for the cultivation of *Bifidobacterium lactis* HN019, wherein the weight ratio of the galactooligosaccharide to lactulose is 1:1 to 4:1.

## Description

This application claims the priority of Chinese patent application No. 201911115800.7 filed with the Chinese Patent Office on November 15, 2019, titled as "SYNERGISTIC BUTYRIC-ACID-PRODUCING COMBINATION OF PREBIOTICS AND PROBIOTICS", the entire content of which is incorporated in this application by reference.

### FIELD

The present invention relates to the field of foods, dietary supplements and medicaments, in particular to a composition comprising probiotics and prebiotics, a food, dietary supplement or pharmaceutical preparation comprising the composition, and a use of the composition for alleviating, ameliorating a disease or discomfort, or reducing the occurrence of a disease or discomfort, wherein the disease or discomfort is associated with an insufficiency of butyric acid in the intestine, or an insufficiency of butyric acid-producing microorganisms in the intestine. The *present invention* also provides a method for promoting the production of butyric acid by *Bifidobacterium lactis* HN019 by using galactooligosaccharide and lactulose as carbon sources for culturing *Bifidobacterium lactis* HN019, wherein the weight ratio of galactooligosaccharide to lactulose is 1:1-4:1.

### BACKGROUND

The human intestinal system contains a large number of bacteria, the total number of which is more than 100 times that of the human cells in the body. These bacteria constitute the human intestinal microbiota. More and more studies have pointed out that the intestinal microbiota is inseparable for the health of the human body, especially the intestinal microbiota in early life has an important impact on lifelong health (Sommer F, Bäckhed F. Nat Rev Microbiol. 2013; 11(4):227-38).

Available studies have pointed out that different feeding methods have an important impact on the early intestinal microbiota of infants. The proportion of bifidobacteria and lactobacilli in the feces of infants in the formula-fed group was significantly lower than that in the infants in the breast-fed group. By adding probiotics and prebiotics to infant formula milk powder, the intestinal microbiota of infants can be regulated, so that the intestinal microbiota composition of formula-fed infants is closer to that of breast-fed infants, especially the proportion of bifidobacteria (Adlerberth I, Wold AE. ActaPaediatr. 2009; 98:229-38).

Prebiotics are a class of indigestible carbohydrates that can interact with intestinal flora in the host intestine to produce substances or effects that are beneficial to the host (Gibson GR et al. Nat Rev Gastroenterol Hepatol. 2017; 14(8):491-502). Prebiotics that have been studied in detail at present include: galactooligosaccharide, fructooligosaccharide, inulin, lactulose, polydextrose, xylo-oligosaccharides, etc.

Galactooligosaccharide (GOS) is a functional oligosaccharide with natural properties, and generally, its molecular structure is linking one or more galactosyl groups to galactosyl or glucose groups. Animal milk contains a small amount of GOS, and human breast milk contains a little more GOS. GOS has an important physiological role in the establishment of intestinal microbiota in animals and lactating infants.

Lactulose (LOS) is the first discovered prebiotics (Ackerman DL et al.Carbohydr Res. 2017; 437:16-27). Before the concept of "prebiotics" came out, lactulose was called "bifido factor", which had a good effect on promoting the growth of bifidobacteria. Lactulose is a disaccharide with various physical and chemical advantages of low sweetness, good acid resistance and thermal stability. Lactulose has prebiotic, laxative and detoxifying effects in different dosages. Watson's *in vitro* results show that lactulose has a good growth-promoting effect on most *Bifidobacterium* and *Lactobacillus* probiotics (Hill C et al. Nat Rev Gastroenterol Hepatol. 2014; 11(8):506-514). The Scientific Opinion of European Food Safety Authority (EFSA) Directive 224/2006, Article 13(1) on substantiation of health claims states that lactulose helps reduce potentially pathogenic microorganisms and reduce the intestinal transit time. Lactulose is metabolized by probiotics to produce short-chain fatty acids such as lactic acid and butyric acid, which is beneficial to the growth and metabolism of intestinal epidermal cells, and can inhibit the growth of harmful intestinal bacteria by reducing the pH value (Dhiren Pranamiet al. Drugs Ther Perspect. 2017; 33:228-233).

The World Food and Agriculture Organization (FAO) and the World Health Organization (WHO) define probiotics as: live bacteria capable of exerting an effective effect on the health of the consumer by ingesting appropriate quantities (Hill Cet al. Nat Rev Gastroenterol Hepatol. 2014; 1(8):506-514). *Bifidobacterium lactis* HN019 (Sanders ME et al. J.Clin.Gastroenterol. 2006; 40(9):776-783) is a probiotic strain isolated from dairy products, which has strong survival ability in the intestine, and can effectively promote intestinal peristalsis. It is a strain that can significantly reduce the intestinal transit time at the lowest concentration among the currently published *Lactobacillus*/*Bifidobacterium* strains (Waller PAet al. Scand J Gastroenterol. 2011; 46(9): 1057-1064). Clinical studies have shown that HN019 can promote the absorption of nutrients in children and improve the immunity of the elderly.

It has been a research hotspot to optimize the combination of probiotics and prebiotics develop an infant formula to make the intestinal micrbiota composition of formula-fed infants more closely resemble that of breast-fed infants.

### SUMMARY

Carbohydrates that cannot be directly utilized by the human body are fermented by gut bacteria to produce short-chain fatty acids (SCFAs), mainly including lactic acid (i.e. 2-hydroxypropionic acid), acetic acid, propionic acid and butyric acid. These short-chain fatty acids can have a positive effect on the health of the human body.

The inventors have surprisingly found that the combination of a specific ratio of galactooligosaccharide and lactulose can synergistically promote the production of butyric acid by *Bifidobacterium lactis* HN019, which can be used to reduce the occurrence of diseases or discomforts associated with an insufficiency of butyric acid in the intestine or an insufficiency of butyric acid-producing microorganisms in the intestine, or be used to alleviate, ameliorate a disease or discomfort associated with an insufficiency of butyric acid in the intestine or an insufficiency of butyric acid-producing microorganisms in the intestine.

In one aspect, the present invention provides a composition comprising galactooligosaccharide, lactulose and *Bifidobacterium lactis* HN019, wherein the weight ratio of galactooligosaccharide to lactulose is 1:1- 4:1.

In one aspect, the application provides a food or dietary supplement comprising the composition. In certain embodiments, the composition, food or dietary supplement of the present invention is used to alleviate, ameliorate a disease or a discomfort, or to reduce the occurrence of a disease or a discomfort, and the disease or discomfort is associated with an insufficiency of butyric acid in the intestine, or an insufficiency of butyric acid-producing microorganisms in the intestine.

In one aspect, the present invention provides a pharmaceutical preparation, wherein the pharmaceutical preparation comprises a composition of the present invention and one or more pharmaceutically acceptable carriers.

In one aspect, the present invention provides a method of alleviating, ameliorating a disease or discomfort, or reducing the occurrence of a disease or discomfort, and the disease or discomfort is associated with an insufficiency of butyric acid in the intestine, or an insufficiency of butyric acid-producing microorganisms in the intestine, and the method includes administering a composition, food or dietary supplement of the present invention to an individual in need thereof.

In another aspect, the present invention provides a use of the composition of the present invention for the preparation of a food, dietary supplement or medicament, and the food, dietary supplement or medicament can be used to alleviate, ameliorate a disease or discomfort, or to reduce the occurrence of a disease or discomfort, and the disease or discomfort is associated with an insufficiency of butyric acid in the intestine, or an insufficiency of butyric acid-producing microorganisms in the intestine.

The present invention also provides a method for promoting the production of butyric acid by *Bifidobacterium lactis* HN019 by using galactooligosaccharide and lactulose as a carbon source for culturing *Bifidobacterium lactis* HN019, wherein the weight ratio of galactooligosaccharide to lactulose is 1:1-4:1.

The present invention also provides a composition for promoting the production of butyric acid by *Bifidobacterium lactis* HN019, and the composition comprises galactooligosaccharide and lactulose, wherein the weight ratio of galactooligosaccharide to lactulose is 1:1-4:1.

### Composition comprising prebiotics and probiotics

In one aspect, the present invention provides a composition comprising galactooligosaccharide, lactulose and *Bifidobacterium lactis* HN019, wherein the weight ratio of galactooligosaccharide to lactulose is 1:1-4:1 (e.g. 1:1-2:1, 2:1-3:1 or 3:1-4:1, the unit of the weight ratio may be g:g).

In certain embodiments, the composition comprises about 10⁶ Cfu/100g - about 10¹² Cfu/100g (e.g. about 10⁶Cfu/100g - about 10⁷Cfu/100g, about 10⁷Cfu/100g - about 10⁸Cfu/100g, about 10⁸Cfu/100g - about 10⁹Cfu/100g, about 10⁹Cfu/100g - about 10¹⁰Cfu/100g, about 10¹⁰Cfu/100g - about 10¹¹Cfu/100g or about 10¹¹Cfu/100g - about 10¹²Cfu/100g) of *Bifidobacterium lactis* HN019.

In the composition of the present invention, galactooligosaccharide (GOS) may be beta-galacto-oligosaccharides and/or alpha-galacto-oligosaccharides. The structure of galactooligosaccharide can be represented by the following formula: [galactose]ₘ-glucose, or represented by the following formula: [galactose]ₙ, wherein the m and n are each independently selected from 2, 3, 4, 5, 6, 7, 8, 9 or 10.

Exemplary galactooligosaccharide structures are 1-7 galactosyl groups attached to the galactosyl side of lactose primarily via β-1,4-glycosidic linkages. In an exemplary galactooligosaccharide structure, the glucose group and the galactosyl group are connected via β-1,4-glycosidic bonds, and the adjacent galactosyl groups are linked by β-1,3, β-1,4 and β-1,6-glycosidic linkages, wherein the β-1,4-glycosidic bond is the main one. The common galactooligosaccharides are mainly galactooligotrisaccharide, galactooligotetrasaccharide and a small amount of GOS5, GOS6, etc. In addition, the β-D-galactosidase of some microorganisms can catalyze glucose and galactose produced from the hydrolysis of the substrate lactose, to synthesize galactooligodisaccharides linked by β-1,3 and β-1,6-glycosidic bonds, which are structurally different from lactose. The molecular structure of an exemplary galactooligosaccharide is shown below:

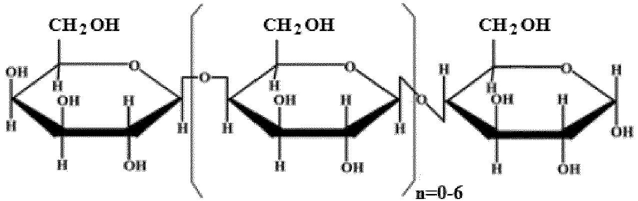

Commercially produced galactooligosaccharides are usually mixtures of glucose, galactose, lactose, galactooligodisaccharide (GOS2), galactooligotrisaccharide (GOS3), galactooligotetrasaccharide (GOS4), GOS5, GOS6, GOS7 and/or GOS8. Therefore, in the present invention, "galtooligosaccharides" or "GOS" may also refer to mixtures of galactooligosaccharides with different chain lengths (e.g. a mixture comprising glucose, galactose, lactose, galactooligodisaccharide (GOS2), galactooligotrisaccharide (GOS3), galactooligotetrasaccharide (GOS4), GOS5, GOS6, GOS7 and/or GOS8).

In certain embodiments, the galactooligosaccharides used in the present invention are galactooligosaccharides that meet the quality requirements in the "Announcement on Approval of New Resource Foods such as Galactooligosaccharides" issued by the Ministry of Health in 2008. In certain embodiments, the galactooligosaccharide is a colorless transparent or light yellow syrup, wherein, on a dry basis, the content of galactooligosaccharides (GOS2 to GOS8) is not less than 57.0%, the content of anhydrous lactose is not higher than 23.0%, the content of anhydrous glucose is not higher than 22.0%, the dry matter is 74.0%-76.0%, and the pH is 2.8-3.8.

The galactooligosaccharide used in the present invention may be a lactose-derived galactooligosaccharide. In certain embodiments, the galactooligosaccharide is obtained as follow: using lactose in cow's milk as a raw material, catalyzed by β-galactosidase to hydrolyze galactosidic bonds to generate galactose and glucose, and then the hydrolyzed galactosides are transferred to lactose molecules through the action of transgalactosidation to generate galactooligosaccharides.

In the composition of the present invention, lactulose is also called 4-β-D-galactoside-D-fructose, 4-O-β-D-galactopyranosyl-4-D-fructose, which can be used in solid or liquid form. In certain embodiments, lactulose is used in the present invention in the form of solid powder, for example, solid products that meets the national food safety standard of GB1886176-2016, wherein the content of lactulose is not less than 97%. In certain embodiments, lactulose is used in the present invention in liquid form, for example, the lactulose liquid that meets the national food safety standard of GB1886176-2016, wherein the content of lactulose is not less than 50%.

In the composition of the present invention, *Bifidobacterium lactis* HN019, also known as *Bifidobacterium animalis* subsp. *lactis* HN019, is a Gram-positive, non-motile, non-spore-forming, irregular rod-shaped anaerobic bacterium with an optimum growth temperature of 37-41°C. It can ferment a variety of sugars, but not starch, can grow in milk or milk-containing media, and is a commercial strain that has been used publicly.

In the present invention, *Bifidobacterium lactis* HN019 may be provided as living cells or non-living cells, and may be in various forms, for example, freeze-dried cells, wet cells, culture broth, yoghurt or a combination thereof. In certain embodiments, lyophilized cells are preferred. *Bifidobacterium lactis* HN019 is commercially available.

The compositions of the present invention may be in liquid, semi-liquid, powder or other form. In certain embodiments, the composition further comprises excipients or dispersants.

In certain embodiments, in the composition, galactooligosaccharide and lactulose together comprise at least 50% by weight of the composition, such as at least 60%, at least 70%, at least 80%, at least 90%, at least 95% or at least 99%. In certain embodiments, the composition consists of galactooligosaccharide, lactulose and *Bifidobacterium lactis* HN019.

The compositions of the present invention may be added into foods as synbiotics, synbiotic combinations or synbiotic mixtures or alone as dietary supplements. The synbiotics, synbiotic combinations or synbiotic mixtures all refer to combinations of probiotics and prebiotics, which can enhance the growth and reproduction ability of probiotics and the survival ability in the host intestinal system, thereby bringing health benefits to the host.

### Food, dietary supplement and uses thereof

In another aspect, the present invention provides a food, and the food comprises the composition of the present invention.

The food of the present invention may be a liquid food, a semi-liquid food, a solid food or a semi-solid food.

In certain embodiments, the food of the present invention is a formula, such as an infant formula (e.g. infant formula, follow-up formula, toddler formula, infant formula for special medical purposes).

In the present invention, the "formula" refers to a nutritional composition designed for infants, toddlers, children, adults, or a combination thereof, containing sufficient nutrients (e.g. protein, carbohydrates, lipids, vitamins, minerals and electrolytes) that may serve as supplemental, primary or sole nutritional sources.

In the present invention, the "infant" refers to a person up to about 12 months of age including infants from 0 to about 4 months old, infants from about 4 to about 8 months old, and infants from about 8 to about 12 months old.

In the present invention, the "older infant" refers to a person between about 6 and about 12 months of age.

In the present invention, the "young children" refers to a person older than 12 months and under 3 years old.

In the present invention, the "children" refers to a person over 3 years old and under 12 years old.

In the present invention, the "infant formula" refers to a human milk replacement or substitute suitable for infant consumption, and its energy and nutritional components can meet the normal nutritional needs of infants aged 0-6 months.

In the present invention, the "follow-up formula" refers to formula suitable for 7-12 month-old infants, and its nutritional components can meet part of the nutritional needs of normal older infants, and can be used as a replacement or substitute for human milk.

In the present invention, the "toddler formula" refers to formula suitable for 13-36 month-old infants, and its nutritional components can meet part of the nutritional needs of normal infants, and can be used as a replacement or substitute for human milk.

In the present invention, the "infant formula for special medical purposes" refers to formula designed for the nutritional needs of infants with special medical conditions such as special disorders, diseases or medical conditions; under the guidance of doctors or clinical nutritionists, when eaten alone or in combination with other foods, its energy and nutrients can meet the growth and development needs of infants with special medical conditions aged 0 to 6 months.

In certain embodiments, in addition to the composition of the present invention, the infant formula (e.g. infant formula, follow-up formula, toddler formula or infant formula for special medical purposes) further comprises the following ingredients: proteins (e.g. milk protein, soy protein), fats (e.g. linoleic acid, alpha-linolenic acid) and carbohydrates (e.g. lactose, lactose and glucose polymers) as energy sources, vitamins (e.g. vitamin A, vitamin D, vitamin K, vitamin B1, vitamin B2, vitamin B6, vitamin B12, niacin, folic acid, vitamin C, biotin, choline), minerals (e.g. sodium, potassium, copper, magnesium, iron, zinc, manganese, calcium, phosphorus, iodine, chlorine, selenium), optionally, the infant formula may further comprise inositol, taurine, L-carnitine, docosahexaenoic acid, eicosatetraenoic acid, or any combination thereof.

In certain embodiments, the formula of the present invention is a liquid or powdered product produced and processed by physical methods only.

In certain embodiments, the food of the present invention is an infant food supplement.

In certain embodiments, the food of the present invention is a dairy product (e.g. 1st stage formula milk powder, 2nd stage formula milk powder, 3rd stage formula milk powder, fermented milk), chocolate or chocolate product, a grain product (e.g. dry cereal mixes, cereal bars, porridge products) or a pet food.

In another aspect, the application provides a dietary supplement, and the dietary supplement comprises the composition of the present invention.

The dietary supplement of the present invention may be in the form of pills, capsules, tablets, granules, liquids or other forms.

In addition to the composition, the food or dietary supplement of the present invention may optionally be supplemented with nutritional ingredients selected from the group consisting of proteins, carbohydrates, lipids, minerals, vitamins, amino acids, unsaturated fatty acids, polyphenols, phytosterols, and a mixture thereof.

The ingredients of the composition of the present invention can be mixed into the other ingredients of the food or dietary supplement sequentially, together or as a premix by conventional processing techniques.

In certain embodiments, the composition, food or dietary supplement of the present invention is used to alleviate, ameliorate a disease or discomfort, or to reduce the occurrence of a disease or discomfort, and the disease or discomfort is associated with an insufficiency of butyric acid in the intestine, or an insufficiency of butyric acid-producing microorganisms in the intestine.

In the present invention, the "insufficiency of butyric acid in the intestine" or "insufficiency of butyric acid-producing microorganisms in the intestine" refers to lower levels of butyric acid or a lower number of butyric acid-producing microorganisms in the gut compared to groups that did not develop the associated disease or discomfort.

Butyric acid has a sour odor, which is unacceptable when it is taken orally. The composition, food, dietary supplement or pharmaceutical preparation of the present invention can be used as a substitute for butyric acid to reduce or alleviate, improve the disease or discomfort related to the insufficiency of butyric acid in the intestine, or the insufficiency of butyric acid-producing microorganisms in the intestine, and can be used safely and conveniently in the population including infants and young children.

In one aspect, the application provides a use of the composition of the present invention for the manufacture of a food, dietary supplement or medicament, and the food, dietary supplement or medicament is used for alleviating, ameliorating a disease or discomfort, or reducing the occurrence of a disease or discomfort, and the disease or discomfort is associated with an insufficiency of butyric acid in the intestine, or an insufficiency of butyric acid-producing microorganisms in the intestine.

The application also provides a pharmaceutical preparation, the pharmaceutical preparation comprises the composition of the present invention and one or more pharmaceutically acceptable carriers.

The pharmaceutical preparation of the present invention may be in any dosage form that is pharmaceutically acceptable. Preferably, the pharmaceutical preparation of the present invention is an oral preparation, for example, an oral solid preparation, such as tablets, capsules, pills, granules, etc.; or, an oral liquid preparation, such as oral solutions, oral suspensions, syrups, etc. The oral preparation may also contain suitable fillers, binders, disintegrants, lubricants, etc.

The "effective amount" of the present invention refers to an amount sufficient to obtain, or at least partially obtain, the desired effect. For example, a "prophylactically effective amount" refers to an amount sufficient to prevent, arrest or delay the onset of a disease or symptom; a "therapeutically effective amount" refers to an amount sufficient to cure or at least partially arrest a disease or symptom in a patient already suffering from the disease or symptom. Determining such effective amounts is well within the ability of a person having ordinary skill in the art. For example, an amount effective for therapeutic use will depend on the severity of the disease or condition being treated, the general state of the patient's own immune system, the general condition of the patient such as age, weight and gender, the mode of administration of the drug, other treatments administered concurrently, etc.

In certain embodiments, the pharmaceutical preparation is used for alleviating, ameliorating a disease or discomfort, or reducing the occurrence of a disease or discomfort, and the disease or discomfort is associated with an insufficiency of butyric acid in the intestine, or an insufficiency of butyric acid-producing microorganisms in the intestine.

In one aspect, the *present invention* provides a method of alleviating, ameliorating a disease or discomfort, or reducing the occurrence of a disease or discomfort, and the disease or discomfort is associated with an insufficiency of butyric acid in the intestine, or an insufficiency of butyric acid-producing microorganisms in the intestine, and the method includes administering a composition, food, dietary supplement or pharmaceutical preparation of the present invention to an individual in need thereof.

In the present invention, the "individual" includes humans or non-human animals. An exemplary human individual includes human individuals (referred to as patient) with diseases or discomforts (e.g. a disease or discomfort described herein) or normal individuals. The "non-human animal" in the present invention includes all vertebrates, for example, non-mammals (e.g birds, amphibians, reptiles), and mammals, for example, non-human primates, livestock and/or domesticated animals (e.g., sheep, dogs, cats, cows, pigs, etc.).

In certain embodiments, the individual is an infant, toddler, child, or adult (e.g., an adult over the age of 60).

In one aspect, the above-mentioned composition, food, dietary supplement or pharmaceutical preparation, and the composition, food, dietary supplement or pharmaceutical preparation is used for alleviating, ameliorating a disease or discomfort, or reducing the occurrence of a disease or discomfort, and the disease or discomfort is associated with an insufficiency of butyric acid in the intestine, or an insufficiency of butyric acid-producing microorganisms in the intestine.

### Method for promoting the production of butyric acid by Bifidobacterium lactis HN019

The present invention also provides a method for promoting the production of butyric acid by *Bifidobacterium lactis* HN019 by using galactooligosaccharide and lactulose as carbon sources for culturing *Bifidobacterium lactis* HN019, wherein the weight ratio of galactooligosaccharide to lactulose is 1:1-4:1 (for example, 1:1-2:1, 2:1-3:1 or 3:1-4:1).

In certain embodiments, the method is used to promote the production of butyric acid by *Bifidobacterium lactis* HN019 *in vitro,* e.g., under *in vitro* culture conditions.

In certain embodiments, the method is performed *in vitro,* including the steps of:
step 1: providing a medium suitable for the growth of *Bifidobacterium lactis* HN019, and the medium uses galactooligosaccharide and lactulose as a carbon source; and
step 2: inoculating *Bifidobacterium lactis* HN019 into the medium of step 1 and cultivating under anaerobic conditions suitable for the growth of *Bifidobacterium lactis* HN019.

In certain embodiments, the medium in step 1 is MRS medium supplemented with cysteine. In certain embodiments, cysteine is added in an amount of 50-500 mg/L, such as 50-100 mg/, 100-200 mg/, 200-300 mg/, 300-400 mg/ or 400-500 mg/L.

In certain embodiments, the total amount of galactooligosaccharide and lactulose in the medium of step 1 is 0.1%-10%, such as 0.1%-1%, 1%-2%, 2%-3%, 3%-4%, 4%-5%, 5%-6%, 6%-7%, 7%-8%, 8% -9% or 9%-10%.

In certain embodiments, the condition in step 2 is an anaerobic condition at 37-41°C.

In certain embodiments, the method is for promoting the production of butyric acid by *Bifidobacterium lactis* HN019 in an individual.

The methods can be used for non-prophylactic or therapeutic purposes as well as for prophylactic or therapeutic purposes.

In certain embodiments, the methods are used to promote the production of butyric acid by *Bifidobacterium lactis* HN019 in an individual and for non-prophylactic or therapeutic purposes.

The present invention also provides a composition for promoting the production of butyric acid by *Bifidobacterium lactis* HN019, wherein the composition includes galactooligosaccharide and lactulose, wherein the weight ratio of galactooligosaccharide to lactulose is 1:1-4:1 (e.g. 1:1-2:1, 2:1-3:1 or 3:1-4:1).

### Beneficial effects

The combination comprising galactooligosaccharide and lactulose of the present invention can effectively promote the production of butyric acid by *Bifidobacterium lactis* HN019. The combination comprising galactooligosaccharide, lactulose and *Bifidobacterium lactis* HN019 can be used in human body to improve human intestinal microbiota, reduce or alleviate, improve the disease or discomfort related to the insufficiency of butyric acid in the intestine, or the insufficiency of butyric acid-producing microorganisms in the intestine.

### DETAILED DESCRIPTION

The embodiments of the present invention will be described in detail below with reference to the examples, but a person having ordinary skill in the art will understand that the following examples are only used to illustrate the present invention, and should not be regarded as limiting the scope of the present invention.

### Example 1 Growth test of Bifidobacterium lactis HN019

*Bifidobacterium lactis* HN019 was purchased from Danisco. The strain was cultured in de Man Rogosa Sharpe (MRS) broth (Difco, Detroit, MD) supplemented with 0.5 g/LL-cysteine and incubated for 24 h at 37°C under anaerobic conditions. Subsequently, the cultures were passaged twice in semi-synthetic MRS (sMRS) medium supplemented with 0.5 g/LL-cysteine and 1% (w/v) filter sterilized glucose as the sole carbohydrate source.

After the second passage, the culture was prepared for use as inoculum for the growth tests described below. Various carbon source stock solutions were sterilized by passing through 0.22 micron filters and used at 1% final concentration.

HN019 was inoculated into different medium at an amount of OD600 reading of 0.1. MRS+0.05% cysteine medium was supplemented with 1% galactooligosaccharide (GOS), 1% lactulose (LOS) and 1% GOS/LOS composition, respectively. The contents of lactic acid, acetic acid, propionic acid and butyric acid in the above-mentioned various combined culture broths were measured by high performance liquid chromatography (HPLC) at 12 hours.

As shown in Table 1, when using GOS/LOS 1:1 and 4:1 as carbon sources, the production of butyric acid was significantly higher than that of GOS or LOS alone as the carbon source. As to the concentrations of other organic acids and the total amount of organic acids, the results of the GOS/LOS combination group were all between the GOS group and the LOS group alone.

**Table 1 Contents of organic acids in the culture medium**

| | Lactic acid | Acetic acid | Propionic acid | Butyric acid | Total amount of organic acid |
|---|---|---|---|---|---|
| LOS | 0.034±0.002 | 0.238±0.002 | ND | ND | 0.272±0.004 |
| GOS | 0.374±0.007 | 1.081±0.014 | ND | 0.084±0.004 | 1.539±0.017 |
| GOS/LOS 1 : 1 | 0.216±0.019 | 0.650±0.080 | ND | 0.248±0.012^{∗}# | 1.115±0.111 |
| GOS/LOS 4 : 1 | 0.176±0.071 | 0.658±0.002 | ND | 0.248±0.009^{∗}# | 1.082±0.078 |
| GOS/LOS 9 : 1 | 0.109±0.003 | 0.699±0.023 | ND | ND | 0.807±0.019 |

| | | | | | |
|---|---|---|---|---|---|
| ^{∗}: vs LOS, p<0.05; #: vs GOS, p<0.05 | | | | | |

Obviously, the above-mentioned embodiments of the present invention are only examples for clearly illustrating the present invention and are not intended to limit the embodiments of the present invention. For a person having ordinary skill in the art, changes or modifications in other different forms can also be made on the basis of the above-mentioned description, and it is impossible to list all the embodiments here. All obvious changes or changes derived from the technical solutions of the present invention are still within the protection scope of the present invention.

## Claims

1. A composition comprising galactooligosaccharide, lactulose and *Bifidobacterium lactis* HN019, wherein the weight ratio of galactooligosaccharide to lactulose is 1:1- 4:1;
preferably, the composition comprises 10⁶ Cfu/100g - 10¹² Cfu/100g of *Bifidobacterium lactis* HN019;
preferably, the composition further comprises an excipient or dispersant;
preferably, in the composition, galactooligosaccharide and lactulose together comprise at least 50% by weight of the composition;
preferably, the composition consists of galactooligosaccharide, lactulose and *Bifidobacterium lactis* HN019.

2. A food comprising the composition according to claim 1;
preferably, the food is a liquid food, semi-liquid food, solid food or semi-solid food;
preferably, the food is a formula, such as an infant formula or infant food supplement;
preferably, the food is a dairy product, chocolate or chocolate product or grain product.

3. The food according to claim 2, wherein the food is used to alleviate, ameliorate a disease or a discomfort, or to reduce the occurrence of a disease or a discomfort, and the disease or discomfort is associated with an insufficiency of butyric acid in the intestine, or an insufficiency of butyric acid-producing microorganisms in the intestine.

4. A dietary supplement comprising the composition according to claim 1;
preferably, the dosage of the dietary supplement is selected from the group consisting of pill, capsule, tablet, granule and liquid preparation;
preferably, the dietary supplement further comprises a nutritional ingredient selected from the group consisting of protein, carbohydrate, lipid, mineral, vitamin, amino acid, unsaturated fatty acid, polyphenol, phytosterol, and a mixture thereof.

5. The dietary supplement according to claim 4, wherein the dietary supplement is used to alleviate, ameliorate a disease or a discomfort, or to reduce the occurrence of a disease or a discomfort, and the disease or discomfort is associated with an insufficiency of butyric acid in the intestine, or an insufficiency of butyric acid-producing microorganisms in the intestine.

6. Use of the composition according to claim 1 for the preparation of a food or dietary supplement, wherein the food or dietary supplement is used to alleviate, ameliorate a disease or a discomfort, or to reduce the occurrence of a disease or a discomfort, and the disease or discomfort is associated with an insufficiency of butyric acid in the intestine, or an insufficiency of butyric acid-producing microorganisms in the intestine.

7. A method for promoting the production of butyric acid by *Bifidobacterium lactis* HN019 comprising using galactooligosaccharide and lactulose as a carbon source for culturing *Bifidobacterium lactis* HN019, wherein the weight ratio of galactooligosaccharide to lactulose is 1:1-4:1;
preferably, the method is used to promote the production of butyric acid by *Bifidobacterium lactis* HN019 *in vitro.*

8. The method according to claim 7, wherein the method is performed *in vitro* and comprising the following steps:
step 1: providing a medium suitable for the growth of *Bifidobacterium lactis* HN019, and the medium comprises galactooligosaccharide and lactulose as a carbon source; and
step 2: inoculating *Bifidobacterium lactis* HN019 into the medium of step 1 and cultivating under anaerobic conditions suitable for the growth of *Bifidobacterium lactis* HN019.

9. A composition for promoting the production of butyric acid by *Bifidobacterium lactis* HN019 comprising galactooligosaccharide and lactulose, wherein the weight ratio of galactooligosaccharide to lactulose is 1:1-4:1.
